(19) Europäisches Patentamt
European Patent Office
Office européen des brevets

(11) Publication number: **0 077 202**
**B1**

(12) # EUROPEAN PATENT SPECIFICATION

(45) Date of publication of patent specification: **09.04.86**

(51) Int. Cl.⁴: **C 07 C 29/03,** C 07 C 29/48, C 07 C 29/50, C 07 C 31/20

(21) Application number: **82305381.4**

(22) Date of filing: **08.10.82**

(54) **Process for hydroxylating olefins using osmium-halogen compound catalysts.**

(30) Priority: **09.10.81 US 310217**

(43) Date of publication of application:
**20.04.83 Bulletin 83/16**

(45) Publication of the grant of the patent:
**09.04.86 Bulletin 86/15**

(84) Designated Contracting States:
**AT BE DE FR GB IT NL**

(56) References cited:
**EP-A-0 053 023**
**GB-A-1 324 763**
**US-A-3 335 174**
**US-A-3 337 635**
**US-A-3 488 394**
**US-A-4 280 924**

**F.A.COTTON et al.: "Advanced inorganic chemistry: A comprehensive text", Fourth Edition, John Wiley & Sons, 1980, pages 916-917, New York (USA);**

**The file contains technical information submitted after the application was filed and not included in this specification**

(73) Proprietor: **Exxon Research and Engineering Company**
**P.O.Box 390 180 Park Avenue**
**Florham Park New Jersey 07932 (US)**

(72) Inventor: **Michaelson, Robert Charles**
**6 West Prospect Street**
**Waldwick New Jersey (US)**
Inventor: **Austin, Richard Graham**
**570 Wyndemere Avenue**
**Ridgewood New Jersey (US)**

(74) Representative: **Dew, Melvyn John et al**
**Esso Chemical Ltd. Esso Chemical Research Centre P.O. Box 1**
**Abingdon Oxfordshire, OX13 6BB (GB)**

**EP 0 077 202 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to processes for hydroxylating olefins in the presence of a specifically defined osmium-halogen compound catalyst.

Processes for the production of glycols such as ethylene glycol, from olefins are well known in the art. For example, it is well known from the technical literature and patents that olefins can be effectively oxidized with a strong oxidizing agent in the presence of catalytic amounts of specific osmium containing compounds, particularly osmium tetroxide, e.g., to their corresponding glycols.

The patent literature directed to osmium containing hydroxylation catalysts describes various osmium oxides used in conjunction with specific oxidants. The primary oxide catalyst employed in these patents is $OsO_4$, a highly volatile (B.P. 130°C) and toxic substance. Ordinarily, the toxic nature of $OsO_4$ alone, while troublesome to some extent, could be dealt with by reasonably economic precautions. However, the combined properties of high volatility and toxicity (human tolerance is 0.002 mg/m$^3$ of air) render this compound extremely dangerous necessitating large capital expenditures in plant safety equipment and design if one attempts to commercialize a process employing this compound as a catalyst. It is for this reason that commercialization of $OsO_4$ based plants has infrequently occurred in the past, if at all. If commercialization is attempted, the aforedescribed capital investment in safety equipment must reduce the profit margin on the products made by these processes.

Accordingly, it would be of extreme economic significance if alternative osmium catalysts could be identified which possess the property of low volatility and/or low toxicity (in relation to $OsO_4$), together with processes for using the same to achieve glycol product selectivity and yield comparable to or better than the conventional $OsO_4$ catalyst.

One step in this direction is described in U.S. Patent No. 3,335,174, which is directed to the use of water hydrolyzable Group Vb, VI-b and VIII metal halides and oxyhalides (e.g. $OsCl_3$) as hydroxylation and esterification catalysts in conjunction with aqueous $H_2O_2$ as an oxidant. However, the process for using this catalyst requires the presence of lower aliphatic hydrocarbon acids such as glacial, formic, acetic and propionic acid as solvents. Under these conditions the reaction times vary from 1/2 to 4 hours, but at the shorter reaction times it is disclosed that substantial amounts of epoxide result. The only yield disclosed is obtained in connection with tungsten hexachloride in Example 1. This yield is extremely low, i.e., 22%, and includes both half-acetate and diol. Thus, among the major disadvantages of the process described in this patent are the low selectivities to diol and the corrosiveness of metal halides in the presence of glacial acids such as acetic acid.

Aside from the aforenoted disadvantages, the above-described process is also disadvantageous in being limited to the use of $H_2O_2$ as the oxidant. This is disadvantageous from an economic standpoint in that this oxidant is expensive and does not form a saleable co-product.

For example, the commercial attractiveness of processes which employ organohydroperoxides to hydroxylate olefins, such as t-butyl hydroperoxide, are dependent on the ability to use or sell the organic alcohol co-product (derived from the hydroperoxide) such as t-butyl alcohol. Thus, provided the market for the co-product alcohol holds up, it is difficult for an $H_2O_2$ oxdant based olefin hydroxylation system to complete successfully with one which employs an organohydroperoxide as the oxidant. Where one wises to use an osmium catalyst in conjunction with an organohydroperoxide, such catalysts have typically always been osmium oxides such as $OsO_4$. The disadvantages of this catalyst have been discussed above. Consequently, while from a marketing standpoint organohydroperoxide based systems are generally superior to $H_2O_2$ based systems, the economics of the former processes (i.e., organohydroperoxide based) could be improved even further if a suitable non-volatile, preferably non-toxic catalyst could be found to replace $OsO_4$.

Notwithstanding the above, it is obvious that the demand for organic alcohol co-products obtained in conjunction with the use of organohydroperoxide can fluctuate with changing market conditions. Consequently, during periods of low demand for organic alcohol co-product, it would be advantageous if one could switch to oxygen as an oxidant in place of organohydroperoxides. Oxygen is abundantly available in the air and is cheaper than $H_2O_2$. However, as with organohydroperoxides, the osmium catalysts known to be useable with oxygen oxidants are predominantly osmium oxides such as $OsO_4$. Consequently, the disadvantages of these catalysts are imparted to the processes which employ them. It would, therefore, also be economically advantageous if a relatively non-volatile, relatively non-toxic osmium catalyst could be employed in conjunction with an oxygen gas containing oxidant to hydroxylate olefins.

Aside from the aforediscussed U.S. Patent 3,335,174 which is limited to the use of $H_2O_2$ as an oxidant, it is believed that no prior art discloses the use of osmium halides, or oxy halides as catalysts to be used in conjunction with organohydroperoxide or oxygen gas containing oxidants for the hydroxylation of olefins. However, the following patents are discussed to provide a general background of osmium oxide catalysts for hydroxylating olefins.

U.S. Patent No. 2,414,385 discloses the use of hydrogen peroxide and a catalytically active oxide, such as osmium tetroxide, dissolved in an essentially anhydrous, non-alkaline, inert, preferably organic, solvent, to convert, by oxidation, unsaturated organic compounds to useful oxygenated products such as glycols, phenols, aldehydes, ketones, quinones and organic acids. The formation of glycols is achieved by

conducting the reaction at temperatures of between several degrees below 0°C and 21°C. Such low reaction temperatures drastically, and disadvantageously, reduce the reaction rate to commercially unacceptable levels. At temperatures greater than 21°C, the formation of aldehydes, ketones and acids is favored.

U.S. Patent No. 2,773,101 discloses a method for recovering an osmium containing catalyst such as osmium tetroxide, by converting it to the non-volatile osmium dioxide form, distilling the hydroxylation product, re-oxidizing the osmium dioxide to the volatile osmium tetroxide, and then recovering the same by distillation. Suitable oxidizing agents used to oxidize olefins, and re-oxidize the osmium dioxide, include inorganic peroxides such as hydrogen peroxide, sodium peroxide, barium peroxide; organic peroxides, such as t-butyl peroxide or hydroperoxide, benzoyl peroxide; as well as other oxidizing agents such as oxygen, perchlorates, nitric acid, chlorine water and the like. As with other methods of the prior art, the above process yields undesirable by-products (see col. 1 line 55) thus reducing the selectivity of the process.

British Patent Specification No. 1,028,940 is directed to a process for regenerating osmium tetroxide from reduced osmium tetroxide by treatment of the latter with molecular oxygen in an aqueous alkaline solution. More specifically, it is disclosed that when osmium tetroxide is used by itself as an oxidizing agent, or as a catalyst in conjunction with other oxidizing agents, to oxidize hydrocarbons the osmium tetroxide becomes reduced, and in its reduced form is less active than osmium tetroxide itself. Consequently, by conducting the oxidation reaction in the presence of an alkaline medium and supplying oxygen to the medium throughout the process, the osmium tetroxide is maintained in a high state of activity. The oxidation products disclosed include not only ethylene glycol from ethylene but also organic acids from such compounds as vicinal glycols, olefins, ketones and alcohols.

U.S. Patent No. 4,255,596 is directed to a process for preparing ethylene glycol in a homogeneous single-phase reaction medium using ethylbenzene hydroperoxide as the oxidizing agent dissolved in ethylbenzene and osmium tetroxide as the catalyst. The pH of the reaction medium is maintained at about 14 by the presence of tetraalkyl ammonium hydroxide. A small amount of water can dissolve beneficially in the medium to reduce by-product formation and improve selectivity to the glycol.

U.S. Patent No. 4,049,724 describes the preparation of glycols from alkenes and from unsaturated alcohols in an aqueous system using osmium tetroxide and specifying stable and water-soluble aliphatic hydroperoxides, such as t-butyl hydroperoxide, while a critical pH of 8 to 12 is maintained by a suitable combination of alkali metal buffering compounds. The preparation of propylene glycol utilizing t-butyl hydroperoxide is exemplified in the patent at a selectivity based on the hydroperoxide of 45%.

Japanese Patent Application No. Sho 54-145604, published November 14, 1979, is directed to a process for hydroxylating olefins in the presence of $OsO_4$, a quaternary ammonium salt such as tetraethyl ammonium bromide, and a peroxide including organoperoxides and $H_2O_2$ as the oxidant.

See also: U.S. Patent No. 3,317,592 (discloses production of acids and glycols using oxygen as oxidant, $OsO_4$ as catalyst at pH 8 to 10); U.S. Patent No. 3,488,394 (discloses hydroxylation of olefins by reacting olefin and hypochlorite in the presence of $OsO_4$); U.S. Patent No. 3,846,478 (discloses reaction of hypochlorite and olefin in an aqueous medium and in the presence of $OsO_4$ catalyst to hydroxylate the olefin); U.S. Patent No. 3,928,473 (hydroxylation of olefins to glycols with $O_2$ oxidant, octavalent osmium catalyst (e.g. $OsO_4$), and borates as promoter); U.S. Patent No. 3,931,342 (discloses a process for recovering glycols from an aqueous solution containing alkali metal borate and osmium compounds (e.g. $OsO_4$)); U.S. Patent No. 3,953,305 (discloses use of $OsO_4$ catalyst for hydroxylating olefins which is regenerated by oxidizing hexavalent osmium with hexavalent chromium and electro-chemically regenerating hexavalent chromium); U.S. Patent No. 4,203,926 (discloses ethylbenzene hydroperoxide as oxidant used in two-phase system to hydroxylate olefins in presence of $OsO_4$ and cesium, rubidium and potassium hydroxides); U.S. Patent No. 4,217,291 (discloses the oxidation of Osmium (III) or (IV) in an ionic complex with oxygen and an alkali metal, ammonium, or tetra (-lower) alkyl ammonium cation to a valency of greater than +5+organohydroperoxides); U.S. Patent No. 4,229,601 (discloses the use of cesium, rubidium and potassium hydroxides as promoters for $OsO_4$ catalyst and t-butyl hydroperoxide oxidant for hydroxylating olefins); and U.S. Patent No. 4,280,924 (discloses a process for regenerating perosmate catalyst, e.g., cesium, rubidium and potassium perosmate).

EP 053023 which is prior art according to Art 54(3) and (4), concerns a process for hydroxylating olefins involving the use of an organic hydroperoxide, certain specified co-catalysts and a catalyst which may be osmium tetroxide, a salt thereof or an osmate, osmium halide are not disclosed.

As emphasized above, none of the aforenoted patents discloses the use of osmium halides or oxy halides as catalysts for the hydroxylation reaction between olefins, and organohydroperoxide or oxygen oxidants.

Accordingly, the search has continued for relatively non-volatile and/or relatively non-toxic osmium compounds capable of catalyzing the hydroxylation reaction between olefins and an oxidant such as organohydroperoxide or oxygen, as well as processes for improving the reaction between olefins and $H_2O_2$ in the presence of osmium halides.

According to this invention a olefin compound is hydroxylated by a process which comprises reacting in admixture at least one olefinc compound having at least one ethylenic unsaturation, water and an oxidant selected from (I) oxygen, and (II) organo hydroperoxide, in the presence of at least one catalyst in a manner and under conditions sufficient to hydroxylate at least one of said ethylenically unsaturated

groups, said catalyst (a) being capable of catalyzing said hydroxylation reaction and (b) being initially present as at least one osmium halide; with the proviso that when oxygen is employed as the oxidant, said reaction is conducted in the additional presence of at least one co-catalyst selected from transition metal halide, transition metal acetate and transition metal benzoate, said transition metal being selected from Cu, Fe, Mn and Mi, preferably copper. Thus in contrast to some prior art processes the process of this invention will be conducted in the absence of $O_sO_4$ and hydrogen peroxide.

Thus, one aspect of the invention may be summarised as providing an improvement in a process for reacting in admixture at least one olefinic compound having at least one ethylenic unsaturation with water and an oxidant selected from organo hydroperoxides and oxygen, the improvement comprising conducting said reaction in the presence of at least one catalyst in a manner and under conditions sufficient to hydroxylate at least one of said ethylenically unsaturated groups, said catalyst (a) being capable of catalyzing said hydroxylation reaction, and (b) being initially present as at least one osmium halide.

In a preferred embodiment at least one olefin is reacted with at least one oxidant, and water in the presence of at least one catalyst optionally.

At least one co-catalyst, under conditions and in a manner sufficient to hydroxylate at least one ethylenically unsaturated group to its corresponding diol group.

The catalysts employed in the process of the present invention comprise compounds containing osmium and halogen. Such osmium-halogen containing compounds include osmium halides and osmium oxy halides, and complexes thereof, (all of the above being referred to herein collectively as osmium-halides) such as those represented by the structural formulae: $Os(X)_n$ (e.g., $OsX_3$, $OsX_4$, and $OsX_5$); $Os(OH)X_3$; $OsOX_4$; $OsOX_5N$; $OsO_3X_2$; $OsONX_4$; $(M)_n'[OsX_6]^{-2}$; $(M)_n'[OsO_2X_4]^{-2}$; $M^{+1}[Os(OH)X_5]^{-1}$; $(M)_n'[OsO_4X_2]^{-2}$; $(M)_n'[OsO_2(OH)X_2]^{-2}$; $(M)_n'[OsNX_5]^{-2}$; and mixtures thereof: wherein X is halogen independently selected from the group consisting of F, Cl, Br and I; n is an integer which can vary from 3 to 5, M is a cation including cations of alkali (e.g., Li, Na, K, Rb, Cs, Fr) metals, alkaline earth metals (e.g., Be, Mg, Ca, Sr, Ba, Ra), ammonium (i.e. $NH_4+$), tetra hydrocarbyl ammonium (e.g. $(R)_4N^+$) and tetra hydrocarbyl phosphonium (e.g. $(R)_4P^+$) said tetra hydrocarbyl groups being as defined in connection with Group 5 co-catalysts discussed below; and n' is a number which is selected in conjunction with the valence of cation M to achieve a neutral complex; preferably n' is 1.

Representative examples of such compounds include $OsF_3$, $OsCl_3$, $OsBr_3$, $OsI_3$, $OsF_4$, $OsCl_4$, $OsBr_4$, $OsI_4$, $OsF_5$, $Os(OH)Cl_3$, $Os(OH)F_3$, $OsOF_4$, $OsOCl_4$, $OsO_3F_2$, $OsONCl_4$, $K_2[OsCl_2Br_2I_2]$, $(NH_4)_2[OsF_6]$, $Ca[OsI_6]$, $Li_2[OsO_2Cl_4]$, $(CH_3CH_2)_4N[Os(OH)Cl_5]$, $(CH_3CH_2)_4P[Os(OH)Br_5]$, $Mg[OsO_4F_2]$, $Na_2[Os(OH)_2Cl_2]$, $Ba[OsCl_5N]$, $K_2[OsNCl_5]$, $K_2[OsNBr_5]$, and mixtures thereof.

The preferred catalysts are those having a boiling point at atmospheric pressure of typically greater than about 130°C, preferably greater than about 150°C, and most preferably greater than about 175°C.

The most preferred catalysts are those represented by the structural formula $OsX_3$ such as $OsCl_3$.

Since the volatility of these osmium catalysts is reduced, preferably substantially, relative to $OsO_4$, the expensive equipment necessary to deal with the volatile and toxic nature of $OsO_4$ can be eliminated or reduced substantially in cost thereby rendering the overall process extremely competitive from an economic standpoint.

The catalysts having the formula $Os(X)_n$ can be prepared by the general methods described in "Advanced Inorganic Chemistry" by Cotton and Wilkinson (hereinafter Cotton and Wilkinson), p. 909 (4th ed. 1980).

Catalysts having the formula $OsOX_4$ and $OsO_3X_2$ can be prepared by the method described in the "J. Inorganic Nuclear Chemistry" by Hepworth and Robinson, Vol. 4, p. 24 (1957).

Catalysts having the formula $Os(OH)X_3$ can be prepared by the method described in "Comprehensive Inorganic Chemistry", Trotman-Dickerson (ed.) vol. 3, p. 1217 (1973).

Catalysts having the formula $OsONX_4$ can be prepared by the method described in "Comprehensive Inorganic Chemistry" described above at vol. 3, p. 1233.

Catalysts having the formula $(M)_n'[OsX_6]^{-2}$ can be prepared by the general method described in Cotton and Wilkinson, p. 919.

Catalysts having the formula $(M)_n'[OsO_2X_4]^{-2}$ can be prepared by the general method described in Cotton and Wilkinson, p. 917.

Catalysts having the formula $M^{+1}[Os(OH)X_5]^{-1}$ can be prepared by the general method described in "Z. Anorg. Allgen. Chem" by Krauss and Wilken (hereinafter Krauss and Wilken) vol. 137, p. 349 (1924).

Catalysts having the formula $(M)_n'[OsO_4X_2]^{-2}$ can be prepared by the method described in Krauss and Wilken, vol. 145, p. 151 (1925).

Catalysts having the formula $(M)_n'[OsO_2(OH)X_2]^{-2}$ can be prepared by the method described in Cotton and Wilkinson, p. 914.

Catalysts having the formula $(M)_n'[OsNX_5]^{-2}$ can be prepared by the method described in "Inorganic Synthesis", by E. G. Rochow, vol. 6, p. 204 (1960).

The disclosures of all of the above references illustrating the methods of preparation of the aforenoted osmium-halide catalysts are herein incorporated by reference.

The osmium-halide catalysts are employed in amounts effective to catalyze the hydroxylation reaction. Thus, while any effective amount of catalyst will suffice, it is preferred that such effective amounts constitute typically from about $1\times10^{-1}$ to about $1\times10^{-8}$ moles, preferably from about $1\times10^{-2}$ to about

$1\times10^{-6}$ moles, and most preferably from about $1\times10^{-2}$ to about $1\times10^{-4}$ moles, of osmium in the osmium-halide catalyst per mole of olefin ethylenic unsaturation to be hydroxylated.

Alternatively, such amounts may be expressed as varying from about 1 to about 10,000, preferably from about 50 to about 1,000, and most preferably from about 200 to about 800 ppm, based on the total weight of liquid reaction medium exclusive of the weight of olefin and any other additives (e.g., buffers) solvent, or co-catalysts if present.

The osmium-halide catalysts are soluble in aqueous and/or organic polar solvent systems described hereinafter and can be dissolved in said systems for addition to the reaction vessel. For example, the osmium-halide catalysts are preferably added to the reaction vessel as a solution having the catalyst dissolved in a water/organic solvent mixture containing excess organohydroperoxide.

When an organo hydroperoxide is used as an oxidant the aforedescribed catalysts can be employed alone or with one or more promoters (also referred to herein as co-catalysts) which increase the reaction rate of the hydroxylation reaction. Such promoters or co-catalysts can be those conventionally employed in conjunction with $OsO_4$ as well as those disclosed in commonly assigned U.S. patent applications by the inventors herein.

For example, suitable promoters or co-catalysts include alkali (e.g., Li, Na, K, Rb, Cs, and Fr), and alkaline earth metal (e.g., Be, Mg, Ca, Sr, Ba and Ra): halides, hydroxides, carboxylated, aryloates, aryolates and pseudo halides; tetra hydrocarbyl ammonium and tetra hydrocarbyl phosphonium: hydroxides, halides, pseudohalides, carboxylates, aryloates, and aryolates; transition metal: halides, pseudohalides, porphyrins, carboxylates, and aryloates; hydrogen halides; alkyl, aryl, aralkyl, and alkaryl halides; Group III-b, (i.e., B, Al, Ga, In, Tl), IV-b (i.e., Si, Ge, Sn, Pb), V-b (i.e., N, P. As, Sb, Bi) and VI-b (i.e., S, Se, Te, Po) halides; and the halogens $F_2Cl_2$, $I_2Br_2$.

More specifically, suitable alkali and alkaline earth metal halide co-catalysts (referred to herein as Group 1 co-catalysts) include the Li, Na, K, Rb, and Cs iodides, bromides, chlorides and fluorides; and Mg, Ca, Sr, and Ba, iodides, bromides, chlorides, and fluorides and mixtures thereof. Preferred Group 1 co-catalysts include the Na, K, Rb, Cs, Mg and Ca halides.

Suitable alkali and alkaline earth metal hydroxide co-catalysts (referred to herein as Group 2 co-catalysts) include LiOH, NaOH, KOH, RbOH, CsOH, $Ca(OH)_2$, $Ba(OH)_2$, $Mg(OH)_2$ and mixtures thereof.

Preferred Group 2 co-catalysts include the Na, K, Rb, Mg and Ca hydroxides.

Suitable alkali and alkaline earth metal: carboxylate aryloate, and aryloate co-catalysts (referred to herein as Group 3 co-catalysts) include those which possess as anions respectively:

(a) carboxylate anions represented by the structural formula:

$$(R_1)\overset{\overset{\displaystyle O}{\|}}{C}-O- \qquad \text{I}$$

wherein $R_1$ can be substituted or unsubstituted: alkyl, typically alkyl of from about 1 to about 10 carbons, preferably about 1 to about 5 carbons and most preferably about 1 to about 3 carbons, or aralkyl, typically aralkyl wherein the aryl group thereof is as defined in connection with Ar of structural formula II below and the alkyl group thereof is as defined immediately above; said $R_1$ substituents including: hydroxyl; halide (i.e., F, Cl, Br, and I); ether groups represented by the structural formulae $-O-R_2$ and $-R_3-O-R_2$ wherein $R_2$ and $R_3$ are independently selected from the group consisting of alkyl, typically about $C_1$ to about $C_{10}$ alkyl, preferably about $C_1$ to about $C_5$ alkyl, and most preferably about $C_1$ to about $C_3$ alkyl; and ester groups represented by the structural formulae:

$$-\overset{\overset{\displaystyle O}{\|}}{C}-O-R_4, \quad -O-\overset{\overset{\displaystyle O}{\|}}{C}-R_4, \quad -R_5-O-\overset{\overset{\displaystyle O}{\|}}{C}-R_4, \quad \text{and} \quad R_5-O-\overset{\overset{\displaystyle O}{\|}}{C}-R_4-,$$

wherein $R_4$ and $R_5$ which may be the same or different are as defined in connection with $R_2$ and $R_3$; and mixtures thereof;

(b) aryloate anions represented by the structural formula:

$$Ar-\overset{\overset{\displaystyle O}{\|}}{C}-O- \qquad \text{II}$$

wherein Ar is selected from the group consisting of substituted and unsubstituted: aryl, typically aryl of from about 6 to about 14 carbons, preferably from about 6 to about 10 carbons (e.g., 6 carbons), and alkaryl, typically alkaryl wherein the alkyl group is from about 1 to about 6 carbons, preferably from about 1 to about 3 carbons, and the aryl group thereof is as defined above, and wherein said substituents on the Ar group are as defined above in connection with $R_1$; and

(c) aryolate anions represented by the structural formula:

$$Ar—O— \qquad III$$

wherein Ar is as described above in connection with structural formula II, and preferably is aryl.

Illustrative examples of such Group 3 co-catalysts include: sodium acetate, potassium acetate, calcium acetate, cesium acetate, magnesium acetate, potassium ethanoate, sodium propanoate, magnesium butanoate, strontium pentanoate, sodium benzoate, potassium benzoate, magnesium benzoate, calcium benzoate, sodium naphthoate, potassium naphthoate, beryllium naphthoate, sodium 4 - (6 - methyl - 2 - naphthyl) pentanoate, potassium 3 - (7 - methyl - 1 - naphtyl) - propanoate, magnesium 2 - (4 - propyl - 1 - benzyl) ethanoate, calcium phenolate, sodium naphtholate, potassium naphtholate, sodium 3-(ethoxy) propanoate, potassium 4-(propoxy carbonyl) butanoate, calcium 3-(propyl carbonyl oxy) propanoate, magnesium 2-(methyl carbonyl oxy methyl) acetate, beryllium 4-(ethoxy carbonyl methyl) butanoate, cesium 4-(ethoxy methyl) benzoate, sodium 3-(propoxy) naphthoate, potassium 4-(ethoxy carbonyl) benzoate, barium 2-(hydroxy) acetate, rubidium 2-chloropropanoate, magnesium 4-bromobenzoate, magnesium phenolate, and mixtures thereof.

Preferred Group 3 co-catalysts include the Na, K, Rb and Cs acetates.

Suitable alkali and alkaline earth metal pseudo halide co-catalysts (referred to herein as Group 4 co-catalysts) include those which possess pseudo halide anions selected from the group consisting of: $SCN^-$, $SeCN^-$, $TeCN^-$, $OCN^-$, and $CNO^-$, and mixtures thereof.

Illustrative examples of such Group 4 co-catalysts include NaSCN, NaSeCN, KSeCN, CsSeCN, NaTeCN, KTeCN, NaOCN, NaCNO, KOCN, KCNO, CsOCN, CsCNO, CsTeCN, $Mg(SeCN)_2$, $Ca(TeCN)_2$, $Ca(OCN)_2$, $Ca(CNO)_2$.

Preferred Group 4 co-catalysts include the Na, K, Rb and Cs thiocyanates.

Tetra hydrocarbonyl ammonium or phosphonium salt co-catalysts (referred to herein as Group 5 co-catalysts) possess a cation and an anion. The respective cations can be represented by the respective structural formula $(R)_4N^+$ and $(R)_4P^+$ wherein R is a hydrocarbyl group independently selected from the group consisting of substituted and unsubstituted: alkyl, typically alkyl having from about 1 to about 30 carbons, preferably from about 1 to about 20 carbons, and most preferably from about 1 to about 10 (e.g. 1—5) carbons, aryl, preferably aryl having from 6 to about 14 carbons, and most preferably from 6 to about 10 carbons, and alkaryl and aralkyl wherein the aryl and alkyl groups thereof are as described immediately above; said R substituents being as defined in connection with the substituents of $R_1$ described above. Accordingly, the term hydrocarbyl is intended to include both substituted and unsubstituted groups, and mixtures thereof. The anion of the Group 5 co-catalysts are selected from the group consisting of hydroxyl, halide, pseudo halide, carboxylate, aryloate and aryolate, said pseudo halide, carboxylate, aryloate, and aryolate anions, being as defined above in connection with the anions of the alkali and alkali metal salt co-catalysts described above.

Illustrative examples of such Group 5 co-catalysts include tetra methyl ammonium bromide, tetra ethyl phosphonium chloride, tetra decyl phosphonium bromide, tetra phenyl ammonium chloride, tetra phenyl phosphonium bromide, dimethyl diethyl ammonium iodide, methyl triethyl phosphonium chloride, tetra butyl ammonium chloride, phenyl trimethyl ammonium bromide, phenyl trimethyl phosphonium chloride, phenyl triethyl ammonium iodide, phenyl triethyl phosphonium chloride, tetra ethyl ammonium hydroxide, tetra butyl ammonium hydroxide, tetra ethyl phosphonium hydroxide, phenyl triethyl ammonium hydroxide, phenyl trimethyl phosphonium hydroxide, tetraethyl ammonium acetate, tetra butyl phosphonium acetate, phenyl triethyl ammonium acetate, phenyl trimethyl phosphonium acetate, tetraethyl ammonium benzoate, phenyl trimethyl phosphonium benzoate, phenyl triethyl ammonium naphthoate, tetra ethyl ammonium phenolate, tetra butyl phosphonium naphtholate, tetra 2-(methoxy) ethyl phosphonium chloride, tetra 4-(propoxy methyl) phenyl ammonium bromide, di 3-(methoxy carbonyl) -propyl -diethyl phosphonium iodide, di 4-(ethyl carbonyloxy) butyl-dimethyl ammonium chloride, tetra 5-(ethoxy carbonyl methyl) pentyl phosphonium bromide, tetra 4-hydroxy butyl ammonium acetate, tetra 3-chloropropyl phosphonium acetate, tetra methyl ammonium thiocyanate, tetra ethyl phosphonium selenio cyanate, tetra (4-methyl phenyl) ammonium chloride, tetra (3 - phenyl - 1 - propyl) phosphonium bromide.

Preferred Group 5 co-catalysts include the unsubstituted tetra lower alkyl (e.g., $C_1$ to $C_5$ alkyl) ammonium hydroxides, iodides, bromides, fluorides, chlorides and acetates.

Transition metal containing co-catalysts (referred to herein as Group 6 co-catalysts) include those having a cation and anion wherein the transition metal cation is selected from the group consisting of cations of Fe, Co, Ni, Cu, V, Cr, Mn, Sc, Ti, Mo, Ru, Rh, Pd, and W, preferably Cu, Fe, Ni, Co and Mn, most preferably Cu, and mixtures thereof.

Anions of the Group 6 co-catalysts include halide, porphyrin (as defined in the Condensed Chemical Dictionary 9th ed. revised by G. Hawley (1977) including benzoporphyrins), pseudo halide, carboxylate and aryloate; said pseudo halide, carboxylate and aryloate anions being as defined generally in connection with the alkali and alkaline earth metal containing co-catalysts and as illustrated by specific examples of suitable anions in conjunction with other co-catalysts described herein.

Representative examples of Group 6 co-catalysts include $FeF_3$, $FeCl_3$, $FeBr_3$, $FeF_2$, $FeCl_2$, $FeBr_2$, $FeI_2$,

$CoCl_2$, $CoF_3$, $CoF_2$, $NiF_2$, $NiBr_2$, $NiI_2$, $NiCl_2$, $CuF_2$, $CuBr_2$, $CuI_2$, $CuF_2$, CuI, CuCl, CuBr, $VF_5$, $VF_4$, $VF_3$, $VF_2$, $VCl_4$, $VCl_3$, $VBr_4$, $VBr_3$, $VI_3$, $CrF_2$, $CrF_3$, $CrF_4$, $CrF_5$, $CrF_6$, $CrCl_3$, $CrCl_4$, $CrBr_3$, $CrBr_4$, $CrI_3$, $MnCl_2$, $MnCl_3$, $MnCl_4$, $MnBr_3$, $MnI_3$, $ScCl_3$, $ScBr_3$, $ScF_3$, $TiCl_4$, $TiBr_4$, $TiF_4$, $MoCl_3$, $Mo_2Cl_{10}$, $MoBr_4$, $Mo_2F_9$, $MoF_6$, $MoF_5$, $RuF_5$, $RuF_3$, $RuF_4$, $RuF_6$, $RuCl_3$, $RuCl_4$, $RuCl_6$, $RuBr_6$, $RhF_3$, $RhF_4$, $RhF_6$, $PdF_2$, $PdCl_2$, $PdBr_2$, $PdI_2$, $WCl_6$, $WBr_5$, $WCl_3$, $WBr_3$, $WI_3$, copper acetate, copper naphthoate, copper benzoate, copper propanoate, iron acetate, iron benzoate, iron naphthoate, copper 4-ethyl benzoate, iron 4-butyl benzoate, nickel acetate, nickel benzoate, nickel naphthoate, copper decanoate, iron hexanoate, iron phthalocyanine, manganese phthalocyanine, copper phthalocyanine, nickel phthalocyanine, and the Fe, Mn, Cu, and Ni porphyrin salts.

Preferred Group 6 co-catalysts include copper bromide, chloride, iodide, and acetate; iron bromide, chloride, iodide and acetate; manganese bromide, chloride, and acetate, and mixtures thereof.

Suitable hydrogen halides (referred to herein as Group 7 co-catalysts) include HF, HCL, HBr and HI. Preferred Group 7 co-catalysts include HI, HBr, and HCl.

Suitable alkyl, aryl, aralkyl and alkaryl halide co-catalysts (referred to herein as Group 8 co-catalysts) include those represented by the respective structural formulae:

$$R'\text{—}(X)_{n}'' \qquad (IV)$$

and

$$(Ar'\text{+}\text{+}X)_{n}'' \qquad (V)$$

wherein R' can be substituted or unsubstituted: alkyl, typically alkyl of from about 1 to about 20, preferably from about 1 to about 10, most preferably from about 1 to about 5 carbons, and aralkyl, typically aralkyl wherein the alkyl group thereof is as defined immediately above and the aryl group thereof is as defined in connection with Ar' below; Ar' is aryl, typically aryl of from about 6 to about 14, preferably 6 to about 10, most preferably 6 carbons, and alkaryl wherein the alkyl and aryl groups thereof are as defined immediately above; X is at least one halogen independently selected from the group consisting of F, Cl, Br and I, and preferably I and Br; n'' is an integer of from about 1 to about 10, preferably from about 2 to about 8, and most preferably from about 2 to about 6; and said R' substituents include hydroxyl.

Representative examples of Group 8 co-catalysts include iodomethane, bromomethane, 1-iodoethane, 1-bromoethane, 1,2-dibromoethane, 1-chloroethane, 1,2-dichloroethane, 1-iodopropane, 1-bromopropane, 1-chloropropane, 2 - iodo - 1 - methylethane, 2 - bromo - 1 - methylethane, 2 - chloro - 1 - methylethane, 1-iodobutane, 2-bromobutane, 1-chlorobutane, 1 - iodo - 1 - methylpropane, 1 - bromo - 1 - methylpropane, 1 - chloro - 1 - methylpropane, 1 - iodo - 1,1 - dimethylethane, 1 - chloro - 1,1 - dimethylethane, 1 - chloro - 1,1 - dimethylethane, phenyliodomethane, phenylchloromethane, phenylbromomethane, 1,2-dichlorobenzene, 2-bromoethanol, 2-chloroethanol, 2-iodoethanol, 1 - phenyl - 2 - iodoethane, 1 - phenyl - 4,4 - dichlorobutane, 1 - (1,2 - dichloroethyl)benzene, 1 - (1 - chloropropyl)-naphthalene *t*-butyl bromide, *t*-butyl iodide and mixtures thereof.

Preferred Group 8 co-catalysts include bromomethane, 1-bromobutane, 1-iodobutane, 1 - bromo - 1,1 - dimethylethane, 1 - iodo - 1,1 - dimethylethane, 2-bromoethanol, and 2-chloroethanol.

Representative examples of suitable Group III-b, IV-b, V-b and VI-b element halides (according to the periodic chart of Cotton and Wilkinson "Advanced Inorganic Chemistry" [3rd ed. 1972]) referred to herein as Group 9 co-catalysts include halides of Al, Ga, In, Tl, Ge, Sn, Pb, P, Si, As, Sb, Bi, S, Se, Te, and Po.

Specific Group 9 element halides include $AlCl_3$, $GaBr_3$, $TiCl_3$, $SiCl_4$, $SiBr_4$, $PI_3$, $PBr_3$, $SbF_5$, $SbBr_3$, $SbI_3$, $BiCl_3$, $BiBr_3$, $AsI_3$, $AsBr_3$, $AsCl_3$, $SeF_4$, $SeCl_4$, $SeBr_4$, $TeF_4$, and mixtures thereof.

Suitable halogen co-catalysts (referred to herein as Group 10 co-catalysts) include $F_2$, $Cl_2$, $Br_2$, and $I_2$.

One or more co-catalysts described in each of the aforenoted Group 1 to 10 co-catalysts can be employed alone or in conjunction with one or more co-catalysts in the same group and/or with one or more of the co-catalysts in the remainder of said groups in any amounts effective to increase the rate of the hydroxylation reaction relative to the rate observed in their absence.

Accordingly, while any effective amount of co-catalysts can be employed, it is contemplated that such effective amounts constitute typically from about 1 to about 10,000 mole percent, preferably from about 50 to about 1,000 mole percent, and most preferably from about 200 to about 500 mole percent, Group I co-catalyst based on the total number of moles of osmium in the osmium-halide catalyst employed, typically from about 1 to about 10,000 mole percent, preferably from about 50 to about 1,000 mole percent, and most preferably from about 200 to about 500 mole percent, Group 2 co-catalyst based on the total number of moles of the osmium in the osmium-halide catalyst employed; typically from about 1 to about 10,000 mole percent, preferably from about 50 to about 1,000 mole percent, most preferably from about 200 to about 500 mole percent, Group 3 co-catalyst, based on the total number of moles of osmium in the osmium-halide catalyst employed; typically from about 1 to about 10,000 mole percent, preferably from about 50 to about 1,000 mole percent, and most preferably from about 200 to about 500 mole percent, Group 4 co-catalyst, based on the total number of moles of the osmium in the osmium-halide catalyst employed; typically from about 1 to about 10,000 mole percent, preferably from about 50 to about 1,000 mole percent, and most preferably from about 200 to about 500 mole percent. Group 5 co-catalyst based on the total number of moles of osmium in the osmium-halide catalyst employed; typically from about 1 to about 10,000 mole percent, preferably from about 50 to about 1,000 mole percent, and most preferably from about 200 to about 500 mole percent, Group 6 co-catalyst based on the total number of moles of the

osmium in the osmium-halide catalyst employed; typically from about 1 to about 10,000 mole percent, preferably from about 50 to about 1,000 mole percent, and most preferably from about 200 to about 500 mole percent, Group 7 co-catalyst based on the total number of moles of osmium in the osmium-halide catalyst employed; typically from about 1 to about 10,000 mole percent, preferably from about 50 to about 1,000 mole percent, and most preferably from about 200 to about 500 mole percent, Group 8 co-catalyst based on the total number of moles of osmium in the osmium-halide catalyst employed; typically from about 1 to about 10,000 mole percent, preferably from about 50 to about 1,000 mole percent, and most preferably from about 200 to about 500 mole percent, Group 9 co-catalyst based on the total number of moles of the osmium in the osmium-halide catalyst employed; and typically from about 1 to about 10,000 mole percent, preferably from about 50 to about 1,000 mole percent, and most preferably from about 200 to about 500 mole percent, Group 10 co-catalyst based on the total number of moles of the osmium in the osmium-halide catalyst.

Preferred combinations of co-catalysts include the use of at least one Group 1 co-catalyst in combination with at least one co-catalyst falling within any one or more co-catalyst Groups 2—9 (e.g., Group 6).

Illustrative examples of suitable co-catalyst combinations include $CuBr_2$ and NaCl; $CuCl_2$ and $NaBr_2$; $FeCl_3$ and NaCl; $CuBr_2$ and tetra ethyl ammonium chloride; $FeCl_2$ and KBr; $FeBr_3$ and CsCl; and CuI and NaBr.

The oxidant which can be employed to oxidize the olefin is an organic hydroperoxide or oxygen (including (molecular) oxygen-containing gases).

The preferred class of oxidants is the organohydroperoxides. Conventional organohydroperoxides include those having the formula:

$$R''OOH$$

wherein R'' is a substituted or unsubstituted: alkyl, typically $C_3$ to $C_{20}$, preferably $C_3$ to $C_{10}$, most preferably $C_3$ to $C_6$ alkyl; aryl, typically $C_6$ to $C_{14}$, preferably $C_6$ to $C_{10}$, most preferably $C_6$ aryl; aralkyl and alkaryl wherein the aryl and alkyl groups thereof are as defined immediately above; cycloalkyl, typically $C_4$ to $C_{20}$, preferably $C_4$ to $C_{10}$, most preferably $C_4$ to $C_8$ cycloalkyl; as well as oxacyclic having 1 to 5 oxygens and preferably 3 to 20 carbons, and azacyclic having 1 to 5 nitrogens and preferably 3 to 20 carbons; and wherein the substituents of said R'' group include halogen, hydroxyl, ester and ether groups.

Representative examples of suitable organohydroperoxides include ethylbenzyl hydroperoxide, t-butyl hydroperoxide, t-amyl hydroperoxide, cumene hydroperoxide, 2 - methyl - 2 - hydroperoxy-methyl propionate 2-methyl-2-hydroperoxy propanoic acid, pyrrolehydroperoxide, furan hydroperoxide, 2-butylhydroperoxide, cyclohexyl hydroperoxide, and 1-phenyl-ethylhydroperoxide.

The most preferred organic hydroperoxides include t-butyl hydroperoxide, ethylbenzylhydroperoxide, and t-amyl hydroperoxide. Frequently these hydroperoxides are made by the molecular oxygen oxidation of the corresponding hydrocarbon which also produces an alcohol as a by-product. For example, when isobutane is oxidized with molecular oxygen there is produced tertiary butyl hydroperoxide and tertiary butyl alcohol. It is not necessary to separate the alcohol from the hydroperoxide since the alcohol can function as a diluent or solvent.

The amount of organohydroperoxide employed is not critical and can vary widely. Generally, the organohydroperoxide is employed in less than stoichiometric requirements, i.e., less than 1:1 molar ratio of organohydroperoxide per mole of ethylenic unsaturation in the olefin to be hydroxylated. Thus, while any amount of hydroperoxide effective to hydroxylate the olefin can be employed, it is preferred that such effective amounts constitute a ratio of moles of ethylenic unsaturation in the olefin to moles of organohydroperoxide of from about 0.5:1 to about 100:1, preferably from about 1:1 to about 20:1 and most preferably from about 2:1 to about 10:1.

While the organohydroperoxide can be added to the reaction mixture in anhydrous form, it is prefered to add the organohydroperoxide as an aqueous solution comprising from about 1 to about 99%, preferably from about 10 to about 90%, and most preferably from about 20 to about 70%, by weight hydroperoxide, based on the weight of the aqueous hydroperoxide solution.

If oxygen or an oxygen-containing gaseous mixture (e.g., containing one or more inert gases such as $N_2$ or air) is employed as the oxidant, it is preferred to also employ at least one Group 6 co-catalyst (described above) in conjunction therewith.

The molar ratio of oxygen to olefin ethylenic unsaturation also can vary widely but for safety reasons it is preferably maintained outside explosive limits, said explosive limits usually being expressed as weight percent ratios.

For example, when hydroxylating ethylene or propylene, if oxygen is in excess, the ratio typically will be about 98 weight percent oxygen or more than 2 percent or less of the olefin based on the total weight of these two reactants. Alternatively, if the olefin is in large excess, the oxygen concentration typically will be about 10 weight percent and about 90 weight percent olefin. When oxygen is in excess, olefin can be added during the reaction as the reaction proceeds. On the other hand, where the olefin is in excess, oxygen can be added during the reaction as the oxygen is consumed.

It is also critical to have water present during the hydroxylation reaction since the water is believed to

contribute one of the oxygen molecules constituting one of the hydroxyl groups in the resulting glycol. The source of this water is not critical. Thus, the water formed in-situ during the reaction between $O_2$ with olefin can contribute to the water content for the reaction. Water can also be added separately, preferably as the solvent for the organohydroperoxide. Consequently, water is provided to, and/or is present, in the initial reaction mixture in at least a stoichiometric molar ratio with the molar amount of ethylenic unsaturation of the olefin to be hydroxylated. Such ratios preferably also are present in the reaction mixture at any given time after start-up. Accordingly, water is present in the reaction mixture at molar ratios of water to olefin ethylenic unsaturation to be hydroxylated in the reaction mixture of from about 1:1 to about 100:1, preferably from about 1:1 to about 50:1, and most preferably from about 1:1 to about 20:1. Such molar ratios typically can be achieved by controlling the amount of water in the reaction mixture (including water formed in-situ) to be from about 1 to about 90 percent, preferably from about 15 to about 85 percent, and most preferably from about 20 to about 60 percent, by weight, based on the total weight of the reaction mixture. Preferably the amount of water employed is less than that which will cause separation of the reaction mixture into an aqueous phase and organic phase although this is not a critical condition.

Olefins which can be hydroxylated in accordance with the present invention contain at least one ethylenic unsaturation and comprise any of the unsaturated aliphatic or alicyclic compounds well known in the art for undergoing such hydroxylation reactions. Typically, such compounds will contain from about 2 to about 20 carbons, preferably from about 2 to about 10 carbons, and most preferably from about 2 to about 5 carbons. Such compounds may be straight or branched chain, mono-olefinic, di-olefinic, or poly-olefinic, conjugated or non-conjugated. They may be substituted with such groups as aryl, preferably aryl of from 6 to about 14 carbons, alkyl, preferably alkyl of from 1 to 10 carbons, or aralkyl and alkaryl wherein the alkyl and aryl portions thereof are as described above, as well as with functional groups such as hydroxyl, carboxyl and anhydride.

Typical of such olefins are those represented by the structural formula:

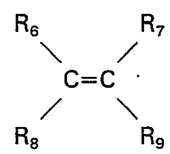

wherein $R_6$, $R_7$, $R_8$, and $R_9$, which may be the same or different, are selected from the group consisting of hydrogen; substituted or unsubstituted: alkyl, aryl, alkaryl, and aralkyl hydrocarbyl groups, said hydrocarbyl groups being preferably as defined immediately above; or any two of said $R_{7-9}$ groups together can constitute a cycloalkyl group typically of from about 4 to about 12, preferably from about 5 to about 8 carbons.

Representative olefins which can be hydroxylated and contain at least one ethylenic unsaturation include: ethylene, propylene, butene-1, butene-2, isobutene, pentene-1, pentene-2, isobutene, butadiene, pentene-1, pentene-2, hexene, isohexene, heptene, 3-methylhexene, octene-1, isooctene, nonene, decene, dodecene, tridecene, pentadecene, octadecene, eicosene, docosene, tricosene, tetracosene, pentacosene, butadiene, pentadiene, hexadiene, octadiene, decadiene, tridecadiene, eicosadiene, tetracosadiene, cyclopentene, cyclohexene, cycloheptene, methylcyclohexene, isopropylcyclohexene, butylcyclohexene, oxtylcyclohexene, dodecyclohexene, acrolein, acrylic acid, 1,2,3,b - tetrahydrophthalic anhydride, methyl methacrylate, styrene, cholesterol, and mixtures thereof.

The preferred olefins are ethylene, propylene, isobutylene, butadiene, styrene, allyl alcohol and allyl chloride.

The most preferred olefin is ethylene and/or propylene.

The preferred mode for conducting the hydroxylation reaction is in a liquid reaction mixture, preferably provided as a homogeneous or substantially homogeneous medium and preferably but optionally by using an inert organic solvent to dissolve or assist in dissolving the osmium and halide containing catalyst, co-catalysts if any, and reactants.

Partial immiscibility of the solvent with water is acceptable although not preferred. By an inert solvent is meant one which does not undergo oxidation during the course of the reaction.

Suitable inert organic solvents preferably possess polar functional groups and include aliphatic or aromatic alcohols having from 1 to about 10 carbon atoms, preferably tertiary alcohols, aliphatic or aromatic ketones having from 3 to about 10 carbon atoms, aliphatic or alicyclic ethers having from 2 to about 10 carbon atoms, glycols having from 2 to about 10 carbon atoms, N,N-dialkyl amides having from 3 to about 10 carbon atoms, nitriles having from about 2 to about 10 carbons, aliphatic or aromatic sulfoxides having from 2 to about 14 carbon atoms, aliphatic or aromatic sulfones having from 2 to about 14 carbon atoms, and the like. Examples of suitable solvents include methanol, ethanol, propanol, butanol, hexanol, decanol, t-butyl alcohol, t-amyl alcohol, benzyl alcohol, acetone, methylethyl ketone, methylbutyl ketone, acetophenone, ethylene glycol, propylene glycol, diethylene glycol, tetraethylene glycol, dimethyl formamide, diethyl formamide, dimethyl acetamide, dimethyl sulfoxide, diethyl sulfoxide, di-n-butyl sulfoxide, diphenyl sulfoxide, dibenzyl sulfoxide, dimethyl sulfone, diethyl sulfone, tetra methylene

sulfone, diphenyl sulfone, acetonitrile, pyridine, dioxane, tetra hydrofuran, tetra hydropyran, dioxolane, and mixtures thereof.

The preferred solvents include those which are substantially or completely miscible with water such as t-butyl alcohol, methanol, and acetonitrile.

The most preferred solvent(s) is the hydroxylated olefin which possesses at least one glycol functionality or mixtures of the product glycol and the product alcohol derived from the organohydroperoxide.

For example, when ethylene is hydroxylated using t-butyl hydroperoxide, the preferred solvent is ethylene glycol or a mixture of ethylene glycol and t-butyl alcohol, the latter being formed in-situ from t-butyl hydroperoxide. The former (product glycol) avoids solvent separation process steps and the latter is economical since the ethylene glycol and t-butyl alcohol are both saleable products which have to be separated anyway. In either instance, an additional solvent separation step is avoided.

The inert solvent is preferably employed in amounts sufficient to achieve a homogeneous solution with respect to at least the olefin and catalyst system. Typically, such amounts can vary from about 0 to about 90 percent, preferably from about 20 to about 80 percent, and most preferably from about 20 to about 50 percent, by weight, based on the total weight of the reaction mixture.

The pH of the reaction mixture during the hydroxylation reaction typically will not be allowed to drop below about 4, preferably not below about 6. Likewise, the pH of the reaction mixture using similar oxidants typically will not be allowed to exceed about 12 although the process can still be conducted at a pH below 4 and above 12. Accordingly, the pH of the reaction mixture typically will be maintained between about 4 and 12, preferably between about 6 and about 12, and most preferably between about 7 and about 12. The pH of the reaction mixture can be controlled by the use of conventional buffers or base where needed. Preferably, pH control is achieved by the use of co-catalyst base.

For example, one mole of HCl may form per mole of $OsCl_3$ catalyst employed under reaction conditions. To compensate for this effect, suitable phosphate, acetate, carbonate, and pyridine buffers can be employed.

In carrying out a preferred embodiment of the invention, olefin, water, oxidant, osmium-halide catalyst, optional co-catalyst, and/or buffer, and optional inert solvent are contacted by admixing to form a liquid reaction medium in a manner and under conditions sufficient to hydroxylate the olefin, i.e., to convert at least one of the ethylenic unsaturations possessed thereby to its corresponding diol. The manner and order of addition of each of the individual components of the liquid reaction medium to the reaction vessel is not critical. However, when an organohydroperoxide is used as the oxidant, it is preferred to mix the osmium-halide catalyst, and optional co-catalyst if employed, with an aqueous solution of the hydroperoxide and then add solvent, additional additives such as buffers, where needed, and finally olefin.

Accordingly, the initial reaction medium prior to introduction of olefin when using an organohydroperoxide oxidant will typically comprise: (a) an organohydroperoxide in an amount of from about 1 to 70 percent, preferably from about 5 to about 60 percent, and most preferably from about 10 to about 50 percent, by weight, based on the weight of the reaction medium exclusive of the weight of olefin, catalyst, and any other additive (e.g. buffers) and/or co-catalysts if present; (b) osmium-halide catalyst in amounts heretofore specified; (c) water, subject to the molar constraints heretofore specified, in an amount of from about 1 to about 98 percent, preferably from about 10 to about 80 percent, and most preferably from about 30 to about 60 percent, by weight, based on the total weight of the reaction medium exclusive of the weight of olefin, catalyst, and any other additives (e.g. buffers) and/or co-catalysts if present; and (d) inert organic solvent in an amount of from about 0 to about 99 percent, preferably from about 20 to about 80 percent, and most preferably from about 30 to about 60 percent, by weight, based on the weight of the reaction medium exclusive of the weight of olefin, catalyst, other additives, and/or co-catalyst if present. Co-catalysts if employed are used in effective amounts as described above as are buffers to control pH where desired.

For the production of ethylene glycol, propylene glycol or any product derived from any unsaturated gaseous olefin, the latter may be bubbled through the reaction mixture containing the components described herein or it may be introduced under pressure. Likewise with the oxygen-containing gas if employed as the oxidant. However, it is preferred that the reaction takes place in the liquid phase. Consequently, sufficient pressure is preferably employed to maintain the gaseous reactants in the liquid phase. Otherwise, the reaction pressure is not critical and can be atmospheric, sub-atmospheric, or super-atmospheric.

When the olefin reactant is a liquid or is dissolved in the reaction mixture under pressure, its concentration in the reaction mixture typically will vary from about 1 to about 98 percent, preferably from about 10 to about 80 percent, and most preferably from about 30 to about 60 percent, by weight, based on the total weight of the reactant mixture inclusive of the weight of components (a) through (d) described above.

The hydroxylation reaction is typically conducted at temperatures which can vary over wide limits although it is preferred to maintain the reaction mixture in the liquid phase. Accordingly, typical reaction temperatures can vary from about 0 to about 250°C, preferably from about 20 to about 150°C, and most preferably from about 30 to about 130°C.

At temperatures greater than the aforenoted ranges, the reaction rate may increase substantially but

this usually occurs at the expensive of a significant reduction in selectivity. At very low reaction temperatures, e.g., below about 0°C the reaction rate decreases to a commercially undesirable degree. Accordingly, while the reaction temperature is not critical and can vary over a wide range, one normally would not operate at temperature extremes outside the aforenoted ranges.

The hydroxylation reaction can be performed as a batch reaction, as a continuous reaction or as a semi-continuous reaction.

In the batch reaction, a reaction medium containing the above described components is charged into the reaction vessel along with olefin if in liquid form. Alterantively, the reaction vessel is then pressurized with olefin if in gaseous form and oxygen if employed. It may be desirable to heat the liquid reaction mixture to reaction temperature prior to pressurizing with the reactant gases. The reaction is allowed to proceed to completion, typically for a period of from about 0.5 to about 5 hours, preferably from about 0.5 to about 3 hours, and most preferably from about 0.5 to about 2 hours.

In the continuous process, the components can be introduced into the inlet of an elongated reactor at a rate such that substantially complete reaction will have taken place by the time the reaction mixture reaches the reactor outlet. The reaction can be carried out in a semi-continuous manner by metering the reactant mixture components into a series of two or more tank reactors at the appropriate rate to maintain the reactor liquid level.

Additionally, the process may be run in either of the aforementioned modes by altering the reaction conditions, and/or, the reactant, solvent, catalyst, co-catalyst, and pH control additive concentrations during the course of the reaction. Thus, the process may be run by changing the temperature, pressure, catalyst concentration, oxidant concentration, and/or olefin concentration.

The spent reaction mixture after removal of unreacted olefin is a solution of product glycol, by-products if any, solvent, water, catalyst and optional co-catalyst. The volatile components are distilled out of the reaction misture into various fractions leaving non-volatile catalyst components in the still. The product glycol is then separated from the high boiling distillate.

In the embodiment wherein an organohydroperoxide, such as t-butyl hydroperoxide, is employed as the oxidant, there is usually no significant amount of unreacted hydroperoxide in the reaction product, since the reaction is generally carried out under conditions, including a stoichiometric excess of olefin, for complete reaction of the oxidant.

If unreacted hydroperoxide is present in the reaction product, it can be removed by the use of a suitable reducing agent in an extra processing step as a safety precaution to avoid possible hazards resulting from the undesired decomposition of the hydroperoxide during product work-up. Therefore, insuring the substantial absence of hydroperoxide in the reaction product represents a safety precaution and avoids substantial processing costs.

The following examples are given as specific illustrations of the claimed invention. It should be understood, however, that the invention is not limited to the specific details set forth in the examples. All parts and percentages in the examples as well as in the remainder of the specification are by weight unless otherwise specified.

Unless otherwise specified, in the following examples selectivity, conversion and yield are calculated as follows:

$$\% \text{ selectivity} = \frac{\text{moles of glycol formed}}{\text{moles of oxygenated product}} \times 100$$

$$\% \text{ conversion} = \frac{\text{moles of product}}{\text{moles of hydroperoxide charged}} \times 100$$

$$\% \text{ yield} = (\% \text{ conversion} \times \% \text{ selectivity}) \div 100$$

Example 1

Into a 100 ml 3-neck round bottom flask equipped with a magnetic stirrer, reflux condenser, dropping funnel, and thermometer, is charged 0.77 g of a 2.5% aqueous solution of $OsCl_3$ (0.1 mmole $OsCl_3$), 18.3 g t-butyl alcohol, 0.76 g (2.9 mmoles) tetra ethylammonium acetate (i.e., $ET_4NOAC$), and 8.0 g (71 mmoles) 1-octene under continuous agitation. An aqueous solution (4.2 g) containing 70% t-butyl hydroperoxide is then added slowly over a period of 45 minutes at 25°C to the mixture thereby introducing a total of 32.7 mmoles of hydroperoxide. The total amount of water in the resulting mixture is 3.7 g. The reaction is allowed to proceed at 25°C and atmospheric pressure, for a period of 12 hours. The contents of the reaction flask are then analyzed by gas chromatography.

The conversion of hydroperoxide is found to be 90 percent. 2.6 g of 1,2-octanediol is obtained indicating a selectivity of 60 percent and a yield of 54 percent.

Example 2

Into a 100 ml 3-neck round bottom flask provided with a magnetic stirrer, reflux condenser, dropping

funnel, and thermometer is charged a sufficient amount of a 0.2% aqueous solution of $OsCl_3$ to introduce 3 mg (0.01 mmole) of $OsCl_3$. Prior to introduction of the $OsCl_3$ aqueous solution into the flask, this solution is premixed in a separate vessel with 0.2 g of t-butyl hydroperoxide added as a 70 percent solution of hydroperoxide in water. Tertiary butyl alcohol (9.6 g) and 1-octene (1.45 g) are then added to the flask and the contents stirred. To this mixture is added slowly, over a period of 10 minutes, a sufficient amount of a 70 percent solution of t-butyl hydroperoxide in water (at 27°C) to introduce 0.90 g of hydroperoxide into the flask. The contents of the flask are warmed to 35°C as a result of the reaction exotherm during the addition of the hydroperoxide solution. The total amount of water present in the flask is 2.0 g. The reaction mixture is stirred continuously and the reaction is allowed to proceed at a temperature of 33—37'C, and atmospheric pressure for a period of 20 minutes measured from completion of the hydroperoxide addition. An additional amount (1.0 g) of the 70 percent aqueous hydroperoxide is then added to the reaction mixture over a period of 5 minutes. The reaction mixture is stirred continuously for an additional 25 minutes.

The contents of the flask are then analyzed by gas chromatography.

The selectivity of the 1,2-octanediol is found to be 52 percent, the conversion is 45 percent, and the yield is 25 percent.

Example 3

Into a 100 ml 3-neck round bottom flask is charged 40.0 mg (0.13 mmole) $OsCl_3$, 25.0 g methanol, and 4.8 g (43 mmole) 1-octene. To the flask is then added slowly, over a period of 20 minutes, a sufficient amount of a 70 percent solution of t-butyl hydroperoxide in water at 25°C to introduce 5.0 g of hydroperoxide thereto. The total amount of water in the flask is 1.5 g. The flask contents are warmed to 27°C by the reaction exotherm after 10 minutes from the start of hydroperoxide addition, and to 45°C after 25 minutes from the start of hydroperoxide addition. The reaction is terminated after 25 minutes from the start of hydroperoxide addition. The contents of the flask are analyzed by gas chromatography. Conversion is found to be 100 percent, selectivity to 1,2-octanediol is 47 percent and the yield is 47 percent.

Example 4

Into a flask as described and equipped in Example 1 is charged, under continuous agitation, t-butyl alcohol (14.0 g) 1-octene (3.0 g) and $OsCl_3$ (0.115 g in 3.7 g $H_2O$). Tetra ethylammonium hydroxide (0.03 g) is added to the flask as a 25 percent by weight solution thereof in methanol. Tertiary butyl hydroperoxide (2.0 g) is added as a 70 percent, by weight, solution thereof in water with stirring over a period of 10 minutes at 25°C. The reaction mixture is warmed to about 46°C during the addition. An additional amount of tetra ethylammonium hydroxide (0.5 g of a 25 percent by weight solution thereof in methanol) is then added to the reaction mixture. The pH of the reaction mixture at this point is about 8. An additional amount of t-butyl hydroperoxide (1.75 g) is then added slowly over a period of 10 minutes at 25°C, and the reaction mixture stirred for an additional 30 minutes. The reaction mixture is then allowed to cool to ambient temperature and analyzed by gas chromatography. The selectivity to 1,2-octanediol is about 82 percent and the conversion is 100 percent.

From the results of the Example 4 it can be seen that the selectivity of the reaction to diol is increased by the presence of base co-catalyst.

Comparative Example 1

The following comparative example is intended to illustrate the effect of the presence of glacial acetic acid in the reaction mixture using $OsCl_3$ catalyst and $H_2O_2$ oxidant in accordance with the general procedure of Example 2 of U.S. Patent No. 3,335,174.

Into a flask, as described and equipped in Example 1, is charged 14.5 ml of a 30 percent, by weight, aqueous solution of $H_2O_2$ and 45 ml of glacial acetic acid and the mixture heated at 75°C for 1 hour. $OsCl_3$ (0.3 g) is then added to the flask and the solution cooled to 28°C. To this solution is added 1-octene (10 ml) over 10 minutes and the solution cooled with an ice bath to maintain the temperature thereof between 20—30°. The pH of the solution is 2. After stirring for an additional 20 minutes, the solution is analyzed by gas chromatography. Conversion of $H_2O_2$ is 10 percent, selectivity to 1,2-octanediol is 10 percent, and selectivity to 1,2-octane diacetate is 20 percent.

Example 5

In a flask, as described and equipped in Example 1, is charged 14.5 ml of a 30 percent $H_2O_2$ aqueous solution, 20.9 g, $H_2O$ and 0.3 g $OsCl_3$. The resulting mixture is stirred for 10 minutes at 25°C. To this solution is then added 10 ml of 1-octene dropwise over a period of 10 minutes. The reaction mixture is cooled with an ice bath to maintain the temperature between 23 and 27°C. The pH of the reaction mixture at the beginning of reaction is 7 and upon completion of the reaction the pH is 5.8. The contents of the flask are then analyzed by gas chromatography. The conversion of $H_2O_2$ is 100 percent, and selectivity to 1,2-octanediol, the major product is about 29 percent.

Comparing the results of Example 5 with comparative Example 1, it can be seen that the absence of glacial acetic acid substantially improves the selectivity and conversion of the reaction. Even greater

improvements can be achieved by maintaining the pH of the reaction mixture during the course of the reaction from neutral to basic.

The principles, preferred embodiments, and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

**Claims**

1. A process for hydroxylating an olefinic compound which comprises reacting in admixture at least one olefinic compound having at least one ethylenic unsaturation, water, and an oxidant selected from (I) oxygen and (II) organo hydroperoxide, in the presence of at least one catalyst in a manner and under conditions sufficient to hydroxylate at least one of said ethylenically unsaturated groups, said catalyst being initially present as at least one osmium halide; with the proviso that when oxygen is employed as the oxidant, said reaction is conducted in the additional presence of at least one co-catalyst selected from transition metal halide, transition metal acetate and transition metal benzoate, said transition metal being selected from Cu, Fe, Mn and Ni.

2. A process according to claim 1 wherein the transition metal of said co-catalyst is copper.

3. A process according to claim 2 wherein said co-catalyst is a copper halide.

4. A process according to any one of the preceding claims wherein said osmium halide is at least one compound represented by a structural formula selected from $Os(X)_n$, $Os(OH)X_3$, $OsOX_4$, $OsOX_5N$, $OsO_3X_2$, $OsONX_4$, $(M)_n'[OsX_6]^{-2}$, $(M)_n'[OsO_2X_4]^{-2}$, $M^{+1}[Os(OH)X_5]^{-1}$, $(M)_n'[OsO_4X_2]^{-2}$, $(M)_n'[OsO_2(OH)X_2]^{-2}$, $(M)_n'[OsNX_5]^{-2}$, and mixtures thereof, in which formulae: X is at least one halide independently selected from F, Cl, Br, and I; n is an integer of from 3 to 5, M is selected from the cations Li, Na, K, Rb, Cs, Fr, Be, Mg, Ca, Sr, Ba, Ra, $NH_4$, tetra hydrocarbyl ammonium, and tetra hydrocarbyl phosphonium; and n' is a number selected in conjunction with the valence of cation M to achieve a neutral complex.

5. A process according to claim 4 wherein the catalyst is one or more of $OsF_3$, $OsCl_3$, $OsBr_3$, and $OsI_3$, preferably $OsCl_3$.

6. A process according to any one of the preceding claims wherein the olefin contains from 2 to 20 carbon atoms per molecule.

7. A process according to claim 6 wherein the olefin is ethylene and/or propylene.

8. A process according to any one of the preceding claims wherein the reaction mixture additionally comprises an inert solvent.

9. A process according to claim 8 wherein the oxidant comprises t-butyl hydroperoxide, the olefin is ethylene, and the inert solvent is selected from t-butyl alcohol, ethylene glycol and mixtures thereof.

10. A process according to any one of claims 1 to 8 wherein the oxidant is at least one organo hydroperoxide.

11. A process according to any one of the preceding claims wherein said reaction is conducted in the presence of at least one co-catalyst selected from alkali metal halide, alkaline earth metal halide, tetra alkyl ammonium carboxylate and tetra alkyl ammonium hydroxide.

12. A process according to any one of claims 1 to 8 wherein the oxidant is oxygen.

13. A process according to any one of the preceding claims wherein the reaction is carried out in the liquid phase.

14. A process according to any one of claims 1 to 12 wherein the hydroxylation reaction is conducted in a homogeneous liquid phase reaction mixture.

**Patentansprüche**

1. Verfahren zur Hydroxylierung einer olefinischen Verbindung, dadurch gekennzeichnet, daß im Gemisch mindestens eine olefinische Verbindung mit mindestens einer ethylenischen Ungesättigtheit, Wasser und ein Oxidationsmittel ausgewählt aus (I) Sauerstoff und (II) Organohydroperoxid in Gegenwart mindestens eines Katalysators in einer Weise und unter Bedingungen umgesetzt werden, welche ausreichen, um mindestens eine der genannten ethylenisch ungesättigten Gruppen zu hydroxylieren, wobei der genannte Katalysator anfänglich als mindestens ein Osmiumhalogenid zugegen ist, mit der Maßgabe, daß dann, wenn als Oxidationsmittel Sauerstoff verwendet wird, die Reaktion in der zusätzlichen Gegenwart mindestens eines Cokatalysators ausgewählt aus Übergangsmetallhalogenid, Übergangsmetallacetat und Übergangsmetallbenzoat durchgeführt wird, wobei das Übergangsmetall ausgewählt ist aus Cu, Fe, Mn und Ni.

2. Verfahren gemäß Anspruch 1, worin das Übergangsmetall des genannten Cokatalysators Kupfer ist.

3. Verfahren gemäß Anspruch 2, worin der genannte Cokatalysator ein Kupferhalogenid ist.

4. Verfahren gemäß einem der vorstehenden Ansprüche, worin das genannte Osmiumhalogenid mindestens eine Verbindung dargestellt durch eine Strukturformel ausgewählt aus $Os(X)_n$, $Os(OH)X_3$, $OsOX_4$, $OsOX_5N$, $OsO_3X_2$, $OsONX_4$, $(M)_n'[OsX_6]^{-2}$, $(M)_n'[OsO_2X_4]^{-2}$, $M^{+1}[Os(OH)X_5]^{-1}$, $(M)_n'[OsO_4X_2]^{-2}$, $(M)_n'[OsO_2(OH)X_2]^{-2}$, $(M)_n'[OsNX_5]^{-2}$ und Gemischen daraus ist, in welchen Formeln X mindestens ein

Halogenid unabhängig ausgewählt aus F, Cl, Br und I bedeutet; n eine ganze Zahl von 3 bis 5 ist, M ausgewählt ist aus den Kationen Li, Na, K, Rb, Cs, Fr, Be, Mg, Ca, Sr, Ba, Ra, $NH_4$, Tetrakohlenwasserstoffammonium und Tetrakohlenwasserstoffphosphonium; und n' eine Zahl ausgewählt im Zusammenhang mit der Wertigkeit des Kations M zur Erzielung eines neutralen Komplexes ist.

5. Verfahren gemäß Anspruch 4, worin der Katalysator eines oder mehrere von $OsF_3$, $OsCl_3$, $OsBr_3$ und $OsI_3$, vorzugsweise $OsCl_3$ ist.

6. Verfahren gemäß einem der vorstehenden Ansprüche, worin das Olefin 2 bis 20 Kohlenstoffatome pro Molekül enthält.

7. Verfahren gemäß Anspruch 6, worin das Olefin Ethylen und/oder Propylen ist.

8. Verfahren gemäß einem der vorstehenden Ansprüche, worin das Reaktionsgemisch zusätzlich ein inertes Lösungsmittel enthält.

9. Verfahren gemäß Anspruch 8, worin das Oxidationsmittel t-Butylhydroperoxid umfaßt, das Olefin Ethylen ist und das inerte Lösungsmittel ausgewählt ist aus t-Butylalkohol, Ethylenglykol und Gemischen daraus.

10. Verfahren gemäß einem der Ansprüche 1 bis 8, worin das Oxidationsmittel mindestens ein Organohydroperoxid ist.

11. Verfahren gemäß einem der vorstehenden Ansprüche, worin die genannte Reaktion in Gegenwart von mindestens einem Cokatalysator ausgewählt aus Alkalimetallhalogenid, Erdalkalimetallhalogenid, Tetraalkylammoniumcarboxylat und Tetraalkylammoniumhydroxid durchgeführt wird.

12. Verfahren gemäß einem der Ansprüche 1 bis 8, worin das Oxidationsmittel Sauerstoff ist.

13. Verfharen gemäß einem der vorstehenden Ansprüche, worin die Umsetzung in flüssiger Phase durchgeführt wird.

14. Verfahren gemäß einem der Ansprüche 1 bis 12, worin die Hydroxylierungsreaktion in einem Reaktionsgemisch aus einer homogenen flüssigen Phase durchgeführt wird.

**Revendications**

1. Procédé d'hydroxylation d'un composé oléfinique, qui consiste à faire réagir en mélange au moins un composé oléfinique ayant au moins une insaturation éthlénique, de l'eau et un oxydant choisi entre (I) l'oxygène et (II) un hydroperoxyde organique, en présence d'au moins un catalyseur d'une manière et dans des conditions suffisantes pour hydroxyler au moins l'un desdits groupes à insaturation éthylénique, ledit catalyseur étant initialement présent sous la forme d'au moins un halogénure d'osmium; sous réserve que lorsque de l'oxygène est utilisé comme oxydant, ladite réaction soit conduite en la présence additionnelle d'au moins un cocatalyseur choisi entre un halogénure de métal de transition, un acétate de métal de transition et un benzoate de métal de transition, ledit métal de transition étant choisi entre Cu, Fe, Mn et Ni.

2. Procédé suivant la revendication 1, dans lequel le métal de transition dudit co-catalyseur est le cuivre.

3. Procédé suivant la revendication 2, dans lequel ledit co-catalyseur est un halogénure de cuivre.

4. Procédé suivant l'une quelconque des revendications précédentes, dans lequel ledit halogénure d'osmium est au moins un composé représenté par une formule structurale choisi entre $Os(X)_n$, $Os(OH)X_3$, $OsOX_4$, $OsOX_5N$, $OsO_3X_2$, $OsONX_4$, $(M)_n'[OsX_6]^{-2}$, $(M)_n'[OsO_2X_4]^{-2}$, $M^{+1}[Os(OH)X_5]^{-1}$, $(M)_n'[OsO_4X_2]^{-2}$, $(M)_n'[OsO_2(OH)X_2]^{-2}$, $(M)_n'[OsNX_5]^{-2}$, et leurs mélanges, formules dans lesquelles: X représente au moins un halogénure choisi indépendamment entre F, Cl, Br et I; n est un nombre entier de 3 à 5, M est choisi entre les cations Li, Na, K, Rb, Cs, Fr, Be, Mg, Ca, Sr, Ba, Ra, $NH_4$, tétrahydrocarbylammonium et tétrahydrocarbylphosphonium; et n' est un nombre choisi conjointement à la valence du cation M pour obtenir un complexe neutre.

5. Procédé suivant la revendication 4, dans lequel le catalyseur est un ou plusieurs des composés $OsF_3$, $OsCl_3$, $OsBr_3$ et $OsI_3$, de préférence $OsCl_3$.

6. Procédé suivant l'une quelconque des revendications précédentes, dans lequel l'oléfine contient 2 à 20 atomes de carbone par molécule.

7. Procédé suivant la revendication 6, dans lequel l'oléfine est l'éthylène et/ou le propylène.

8. Procédé suivant l'une quelconque des revendications précédentes, dans lequel le mélange réactionnel comprend en outre un solvant inerte.

9. Procédé suivant la revendication 8, dans lequel l'oxydant comprend l'hydroperoxyde de tertiobutyle, l'oléfine est l'éthylène et le solvant inerte est choisi entre l'alcool tertio-butylique, léthylèneglycol et leurs mélanges.

10. Procédé suivant l'une quelconque des revendications 1 à 8, dans lequel l'oxydant est au moins un hydroperoxyde organique.

11. Procédé suivant l'une quelconque des revendications précédentes, dans lequel ladite réaction est conduite en présence d'au moins un co-catalyseur choisi entre un halogénure de métal alcalin, un halogénure de métal alcalino-terreux, un carboxylate de tétra-alkylammonium et un hydroxyde de tétra-alkylammonium.

12. Procédé suivant l'une quelconque des revendications 1 à 8, dans lequel l'oxydant est l'oxygène.

13. Procédé suivant l'une quelconque des revendications précédentes, dans lequel la réaction est conduite en phase liquide.

14. Procédé suivant l'une quelconque des revendications 1 à 13, dans lequel la réaction d'hydroxylation est conduite dans un mélange réactionnel à phase liquide homogène.